# EUROPEAN PATENT APPLICATION

(11) **EP 4 218 818 A1**
(43) Date of publication of application: **02.08.2023**
(21) Application number: 21872483.9
(22) Date of filing: 22.09.2021
(51) Int. Cl.: A61K 45/00, A61K 31/428, A61P 25/04, A61P 43/00

(54) **ALPHA-2 ADRENERGIC RECEPTOR ANTAGONIST**

(30) Priority: 23.09.2020 JP 2020158954
(71) Applicant: Kyoto University, Kyoto-shi, Kyoto 606-8501 (JP)
(72) Inventor: HAGIWARA Masatoshi, Kyoto 606-8501 (JP); TOYOMOTO Masayasu, Kyoto 606-8501 (JP)
(74) Representative: Dehns
(86) International application number: PCT/JP2021/034765
(87) International publication number: WO 2022/065354

(57) **Abstract**

Provided in one aspect is a pharmaceutical composition for analgesia. The present disclosure relates to a pharmaceutical composition for analgesia that contains an α2 adrenergic receptor antagonist as an active ingredient. In one or more embodiments, the pharmaceutical composition according to the present disclosure is a composition for analgesia through inhibition of an α2 adrenergic receptor, or a composition for analgesia through inhibition of an α2B adrenergic receptor.

## Description

### Technical Field

The present disclosure relates to an alpha-2 adrenergic receptor antagonist, a composition containing this substance, and use of this substance.

### Background Art

A medical condition of pain is a complicated physiological process that involves several kinds of sensory mechanisms and neural mechanisms. Pain can be defined as an unpleasant sensory or emotional experience accompanied by a real or potential tissue damage, or can be described as such a damage.

Pathophysiologically, pains can be classified into inflammatory pains and neuropathic pains.

The inflammatory pain is a nociceptive pain via nociceptor, which can be considered as a pain caused by an inflammatory mediator released to a site of tissue damage. The mechanism of an inflammatory pain is considered as follows. When a tissue is damaged and inflammation is caused, a pain producing substance such as bradykinin, ATP, or proton, and/or prostaglandin, serotonin, histamine, or an inflammatory cytokine are released, and a spontaneous pain develops incessantly. Further, hyperalgesia occurs due to hypersensitization of a nociceptor. Still further, as a mechanism of hypersensitization of a nociceptor, the phosphorylation of an ion channel, particularly TRPV1 channel known as a capsaicin receptor, is reported.

On the other hand, a neuropathic pain can be defined as a pain that is caused by a lesion or a disease of a somatosensory nervous system (International Association for the Study of Pain in 2011). In Japan, it is estimated that there are several millions of neuropathic pain patients. The pain is a pain that does not involve excitation of a nociceptor, and that involves plastic changes of peripheral nerves or central nerves. As a mechanism of development of a neuropathic pain, the following have been reported: the ectopic firing of peripheral nerves; the neuroanatomical reconstruction in peripheral nerves and dorsal horns of the spinal cord; the control of descending inhibition systems; and the activation of glial cells in the spinal cord.

Several pain-related compounds and pharmaceutical compositions have been reported (Patent Documents 1 and 2).

Adrenergic receptors are G protein-coupled receptors (GPCRs) that are activated by catecholamines such as adrenaline and noradrenaline.

Currently, adrenergic receptors are classified into three types: α1, α2, and β. Each of these is further divided into three subtypes. Subtype α1 includes α1A, α1B, and α1D, subtype α2 includes α2A, α2B, and α2C, and subtype β includes β1, β2, and β3.

GPCRs form the largest superfamily of transmembrane proteins, and transmit signals to the interior of cells in response to extracellular stimulation. Binding of an agonist to the GPCR activates heterotrimeric G proteins constituted by Gα, Gβ, and Gγ subunits and downstream signal transduction dependent on each subunit. In humans, the Gα subunit is encoded by 16 genes and classified into four major Gα families (Gαₛ, Gα_{i/o}, Gα_{q/11}, and Gα_{12/13}), which cause distinct signal transduction cascades.

The TGFα shedding assay is known as an assay by which a signal transduction pathway of a GPCR can be detected and measured (Patent Document 3). When the signal transduction pathway of a GPCR is activated, fusion proteins of membrane-bound alkaline phosphatase (AP) and TGFα are cleaved by TACE (TNFα converting enzyme), which is a membrane protease, and AP-TGFα is released from the cells. This released AP is measured.

### Citation List Patent Document

Patent Document 1: JP-T-2008-539269
Patent Document 2: WO 2015/093567
Patent Document 3: WO2015/128894

### SUMMARY

### Technical Problem

The present disclosure, in one aspect, provides a pharmaceutical composition for analgesia.

The present disclosure, in another aspect, provides a composition for inhibiting an α2 adrenergic receptor.

### Solution to Problem

The present disclosure, in one aspect, relates to a pharmaceutical composition for analgesia that contains an α2 adrenergic receptor antagonist as an active ingredient.

The present disclosure, in one aspect, relates to a composition for inhibiting an alpha-2 adrenergic receptor that contains, as an active ingredient, a compound represented by Formulae (I) to (VIII) below, a prodrug thereof, or a pharmaceutically acceptable salt thereof.

The present disclosure, in one aspect, relates to a composition for analgesia through inhibition of an alpha-2 adrenergic receptor, the composition comprising, as an active ingredient, at least one selected from the group consisting of a compound represented by Formulae (I) to (VIII) below, a prodrug thereof, a pharmaceutically acceptable salt thereof, and a combination thereof.

The present disclosure, in one aspect, relates to a method for inhibiting an α2 adrenergic receptor, the method including administering at least one selected from the group consisting of a compound represented by Formulae (I) to (VIII) below, a prodrug thereof, a pharmaceutically acceptable salt thereof, and a combination thereof.

The present disclosure, in one aspect, relates to a method for relieving pain through inhibition of an α2 adrenergic receptor, the method including administering at least one selected from the group consisting of a compound represented by Formulae (I) to (VIII) below, a prodrug thereof, a pharmaceutically acceptable salt thereof, and a combination thereof.

The present disclosure, in one aspect, relates to use of at least one selected from the group consisting of a compound represented by Formulae (I) to (VIII) below, a prodrug thereof, a pharmaceutically acceptable salt thereof, and a combination thereof, for inhibiting an α2 adrenergic receptor.

The present disclosure, in one aspect, relates to use of at least one selected from the group consisting of a compound represented by Formulae (I) to (VIII) below, a prodrug thereof, a pharmaceutically acceptable salt thereof, and a combination thereof, for relieving pain through inhibition of an α2 adrenergic receptor.

### Advantageous Effects of Invention

The present disclosure, in one or more embodiments, can provide a pharmaceutical composition for providing analgesia.

The present disclosure, in one or more embodiments, can provide a composition for inhibiting an α2 adrenergic receptor.

### Brief Description of Drawings

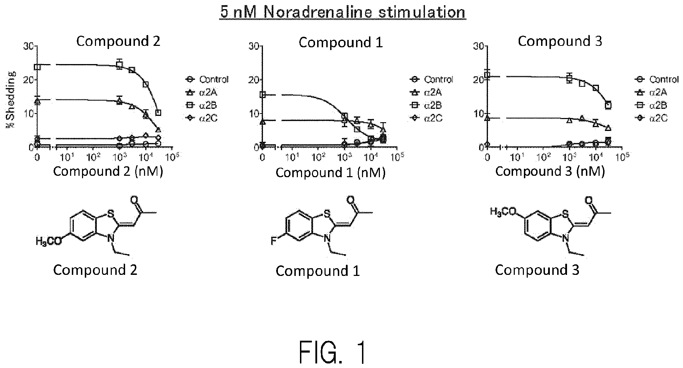

FIG. 1 is an exemplary graph illustrating the results obtained by measuring, through a TGFα shedding assay, the degree of inhibiting signal transduction of α2A adrenergic receptor, α2B adrenergic receptor, and α2C adrenergic receptor in response to noradrenaline stimulation, using compounds 1 to 3.

### Description of Embodiments

The present disclosure is based on the finding that α2 adrenergic receptor antagonists can be used for relieving pain (analgesia).

Details of the mechanism of the present invention are not clear, but it can be presumed as follows. In the descending pain inhibition system, secreted endogenous noradrenaline induces analgesia through receptors. On the other hand, when noradrenaline binds to the α2 adrenergic receptor on presynaptic cells, negative feedback signals are transmitted, and noradrenaline secretion is suppressed. α2 adrenergic receptor antagonists suppress this negative feedback, thereby facilitating activation of the descending pain inhibition system, resulting in pain relief (analgesia).

However, the present disclosure need not be interpreted as being limited to this mechanism.

In the present disclosure, the adrenergic receptor subtype α2 may be denoted as alpha-2 adrenergic receptor, α2 adrenergic receptor, α2-adrenergic receptor, adrenergic receptor α2, or the like. The same also applies to the adrenergic receptor subtypes α2A, α2B, and the like.

In the present disclosure, a receptor antagonist can also be referred to as a compound or composition that inhibits or blocks a receptor. In one or more embodiments, inhibition and blockage includes suppression of the activity of a receptor on which a compound or composition acts, compared to when it does not act on the receptor.

In the present disclosure, analgesia includes suppressing pain, and alternatively, may include reducing pain, controlling the exacerbation of pain, and/or treating pain.

The "pain" in the present disclosure, in one or more embodiments, refers to an inflammatory pain or a neuropathic pain classified pathophysiologically. Further, in the present disclosure, in one or more embodiments, the "pain" refers to an acute pain, an inflammatory pain, a visceralgia, a breakthrough pain, a nociceptive pain, a neuropathic pain, a chronic pain, or a cancer-related pain, which are classified depending on the time lapse or the mechanism.

### [Pharmaceutical composition for analgesia]

The present disclosure, in one aspect, relates to a pharmaceutical composition for analgesia that contains an α2 adrenergic receptor antagonist as an active ingredient.

In one or more embodiments, an α2 adrenergic receptor antagonist is a compound or composition that inhibits an α2 adrenergic receptor, and preferably a compound or composition that selectively inhibits an α2 adrenergic receptor.

The term "selectively inhibits α2 adrenergic receptor " in the present disclosure includes selectively inhibiting α2 adrenergic receptor more effectively than α1 adrenergic receptor and β adrenergic receptor in one or more embodiments.

In one or more embodiments, the α2 adrenergic receptor antagonist is preferably a compound or composition that inhibits the α2B adrenergic receptor, and more preferably a compound or composition that selectively inhibits the α2B adrenergic receptor, from the viewpoint of obtaining an analgesic effect with few side effects.

The term "selectively inhibits α2B adrenergic receptor " in the present disclosure includes selectively inhibiting α2B adrenergic receptor more effectively than α2A adrenergic receptor and α2C adrenergic receptor in one or more embodiments.

In one or more embodiments, the pharmaceutical composition according to the present disclosure can be referred to as a composition for analgesia through inhibition of α2 adrenergic receptor, or a composition for analgesia through inhibition of the α2B adrenergic receptor.

Examples of the α2 adrenergic receptor antagonists of the pharmaceutical composition according to the present disclosure include at least one selected from the group consisting of a compound represented by Formulae (I) to (VIII) below, a prodrug thereof, a pharmaceutically acceptable salt thereof, and a combination thereof, and a composition containing them.

Therefore, the present disclosure, in another aspect, relates to an α2 adrenergic receptor antagonist or an α2B adrenergic receptor antagonist selected from the group consisting of a compound represented by Formulae (I) to (VIII), a prodrug thereof, a pharmaceutically acceptable salt thereof, and a combination thereof.

### [Composition for inhibiting α2 adrenergic receptors]

The present disclosure, in one aspect, relates to a composition for inhibiting an α2 adrenergic receptor. In one or more embodiments, the "composition for inhibiting an α2 adrenergic receptor" in the present disclosure is preferably a composition that selectively inhibits an α2 adrenergic receptor, a composition that inhibits the α2B adrenergic receptor, or a composition that selectively inhibits the α2B adrenergic receptor.

A composition for inhibiting an α2 adrenergic receptor according to the present disclosure, in one or more embodiments, contains a compound represented by Formula (I) below, a prodrug thereof, or a pharmaceutically acceptable salt thereof, as an active ingredient, 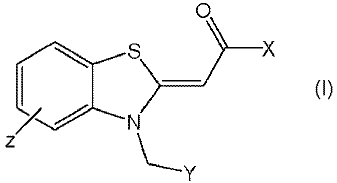 where in Formula (I), X represents an alkyl group, an alkoxy group, a halogen, a hydroxy group, an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, Y represents a hydrogen atom, a halogen, or an alkyl group, and Z represents one to four substituents, in a case where Z represents two or more substituents, the substituents each independently, and the substituent is a hydrogen atom, an alkyl group, an alkoxy group, a halogen, or a hydroxy group.

In Formula (I), in one or more embodiments, X represents an alkyl group, an alkoxy group, a halogen or hydroxy group, preferably a methyl group, an ethyl group, a 1-propyl group, a 2-propyl group, a 2-methyl-1-propyl group, a 2-methyl-2-propyl group, a 1-butyl group or a 2-butyl group, or a methyl group, an ethyl group, a 1-propyl group or a 2-propyl group.

In one or more embodiments, Y represents a hydrogen atom or an alkyl group, preferably a hydrogen atom, a methyl group, an ethyl group, a 1-propyl group, a 2-propyl group, a 2-methyl-1-propyl group, a 2-methyl-2-propyl group, a 1-butyl group or a 2-butyl group, or a methyl group, an ethyl group, a 1-propyl group or a 2-propyl group.

In one or more embodiments, Z represents an alkyl group, an alkoxy group, a halogen, or a hydroxy group, preferably a methyl group, an ethyl group, a 1-propyl group, a 2-propyl group, a methoxy group, an ethoxy group, a 1-propyloxy group, a 2-propyloxy group, a 2-methyl-1-propyloxy group, a 2-methyl-2-propyloxy group or a halogen, or a methoxy group, an ethoxy group, a 1-propyloxy group, a 2-propyloxy group, a 2-methyl-1-propyloxy group, a 2-methyl-2-propyloxy group or a halogen. In one or more embodiments, Z is a substituent at the 5- or 6-position of benzothiazole.

In Formula (I), in one or more embodiments, X represents an alkyl group, an alkoxy group, a halogen or a hydroxy group, Y represents a hydrogen atom or an alkyl group, Z represents an alkyl group, an alkoxy group, a halogen, or a hydroxy group, and preferably, X represents an alkyl group or an alkoxy group, Y represents a hydrogen atom or an alkyl group, and Z represents an alkoxy group or a halogen bonded at the 5- or 6-position of benzothiazole, or X represents an alkyl group, Y represents a hydrogen atom or an alkyl group, and Z represents an alkoxy group or a halogen bonded at the 5- or 6-position of benzothiazole.

In one or more embodiments, the compound represented by Formula (I) is a compound represented by any one of the formulae below.

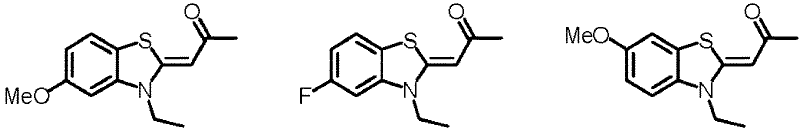

The "alkyl group" in the present disclosure refers to a substituted or unsubstituted straight or branched chain or cyclic alkyl group in one or more embodiments, and substituted or unsubstituted straight or branched chain or cyclic alkyl group having one to six carbon atoms in one or more embodiments.

In the present disclosure, examples of the straight or branched chain alkyl group having one to six carbon atoms, in one or more embodiments, include a methyl group, an ethyl group, a 1-propyl group, a 2-propyl group, a 2-methyl-1-propyl group, a 2-methyl-2-propyl group, a 1-butyl group, a 2-butyl group, a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group, and a 2,3-dimethyl-2-butyl group. Examples of the cyclic alkyl group having one to six carbon atoms, in one or more embodiments, include cyclopropyl, cyclobutyl, cyclopentyl, and cyclohexyl.

In the present disclosure, the "alkoxy group" refers to a substituted or unsubstituted straight or branched chain alkoxy group in one or more embodiments, and substituted or unsubstituted straight or branched chain alkoxy group having one to six carbon atoms in one or more embodiments.

The alkoxy group having one to six carbon atoms in one or more embodiments refers to an oxy group to which the alkyl group having one to six carbon atoms as defined above is bonded, and examples of the alkoxy group include a methoxy group, an ethoxy group, a 1-propyloxy group, a 2-propyloxy group, a 2-methyl-1-propyloxy group, a 2-methyl-2-propyloxy group, a 1-butyloxy group, a 2-butyloxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3-methyl-2-butyloxy group, a 2,2-dimethyl-1-propyloxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butyloxy group, a 3,3-dimetyl-1-butyloxy group, a 2,2-dimetyl-1-butyloxy group, a 2-ethyl-1-butyloxy group, a 3,3-dimetyl-2-butyloxy group, and a 2,3-dimethyl-2-butyloxy group in one or more embodiments.

The substituent in the present disclosure may be one substituent, or a plurality of identical or different substituents, and in one or more embodiments, examples of the substituent include a halogen atom, a cyano group, a trifluoro methyl group, a nitro group, a hydroxyl group, a methylenedioxy group, a C₁₋₆ alkyl group, a C₁₋₆ alkoxy group, a benzyloxy group, a C₁₋₆ alkanoyloxy group, an amino group, a mono-C₁₋₆ alkylamino group, a di-C₁₋₆ alkylamino group, a carbamoyl group, a C₁₋₆ alkylaminocarbonyl group, a di-C₁₋₆ alkylaminocarbonyl group, a carboxyl group, a C₁₋₆ alkoxycarbonyl group, a C₁₋₆ alkylthio group, a C₁₋₆ alkylsulfinyl group, a C₁₋₆ alkylsulfonyl group, a C₁₋₆ alkanoylamino group, and a C₁₋₆ alkyl sulfonamide group.

In the present disclosure, in one or more embodiments, examples of the halogen atom include atoms such as a fluorine atom, a chlorine atom, a bromine atom, and an iodine atom.

A composition for inhibiting an α2 adrenergic receptor according to the present disclosure, in one or more embodiments, contains a compound represented by Formula (II) below, a prodrug thereof, or a pharmaceutically acceptable salt thereof, as an active ingredient, 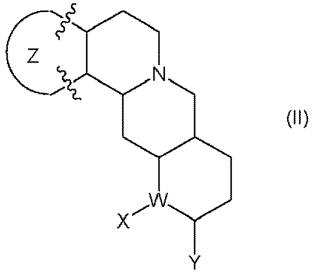 where in Formula (II), W represents CH or a nitrogen atom, X represents an alkoxycarbonyl group, a carboxyl group, an alkylsulfonyl group, an alkylsulfinyl group, a sulfonyl group, a sulfinyl group, a sulfonic acid alkyl ester group, a sulfinic acid alkyl ester group, or a hydrogen atom, Y represents a hydroxyl group, an alkoxy group, a halogen, or a hydrogen atom, and Z represents a fused ring or any one of the following: 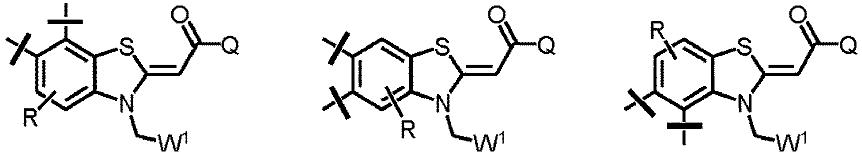 Q represents an alkyl group, an alkoxy group, a halogen, or a hydroxy group, R represents one or two substituents, and in a case where R represents two substituents, the two substituents each independently represent a hydrogen atom, an alkyl group, an alkoxy group, a halogen, or a hydroxy group, and W¹ represents a hydrogen atom, a halogen, or an alkyl group.

Examples of a ring formed as the fused ring include a benzene ring, an alkyl benzene ring, an alkoxy benzene ring, a halogenyl benzene ring, an indole ring, a pyrrole ring, a pyrrolidine ring, an imidazole ring, a pyridine ring, a pyrazine ring, a pyrimidine ring, and a benzothiazole ring.

In one or more embodiments, examples of the compound represented by Formula (II) include compounds represented by the formulae below.

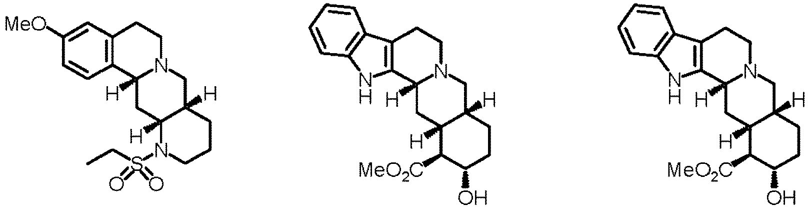

A composition for inhibiting an α2 adrenergic receptor according to the present disclosure, in one or more embodiments, contains a compound represented by Formula (III) below, a prodrug thereof, or a pharmaceutically acceptable salt thereof, as an active ingredient, 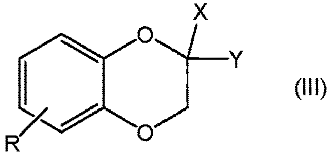 where in Formula (III), R represents one to four substituents, in a case where R represents two or more substituents, the substituents each independently, and the substituent is a hydrogen atom, an alkyl group, an alkoxy group, a halogen, or a hydroxy group, X represents an alkyl group, an alkoxy group, or a hydrogen atom, and Y represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following: 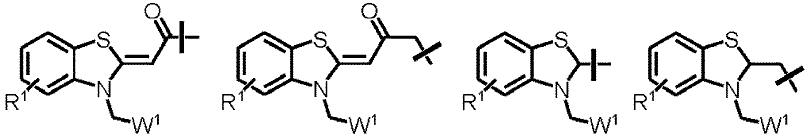 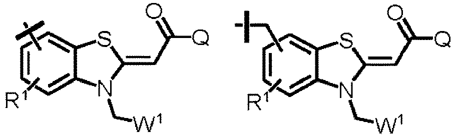 Q represents an alkyl group, an alkoxy group, a halogen, or a hydroxy group, R¹ represents one to three or one to four substituents, in a case where R¹ represents two or more substituents, the substituents each independently, and the substituent is a hydrogen atom, an alkyl group, an alkoxy group, a halogen, or a hydroxy group, and W¹ represents a hydrogen atom, a halogen, or an alkyl group.

In one or more embodiments, examples of the compound represented by Formula (III) include compounds represented by the formulae below.

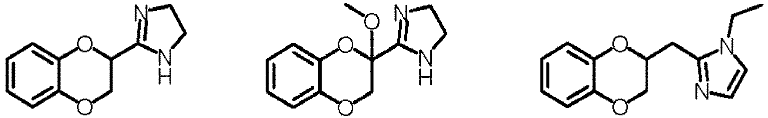

A composition for inhibiting an α2 adrenergic receptor according to the present disclosure, in one or more embodiments, contains a compound represented by Formula (IV) below, a prodrug thereof, or a pharmaceutically acceptable salt thereof, as an active ingredient, 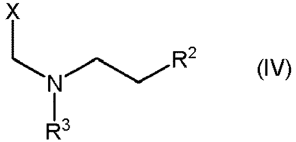
where in Formula (IV), X represent any one of the following: 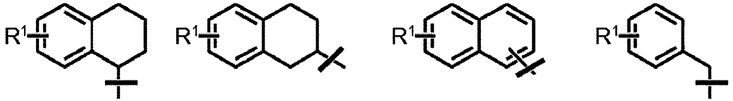
R² represents an indole group, a quinoline group, or a quinazoline group, or any one of the following: 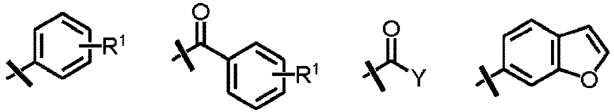 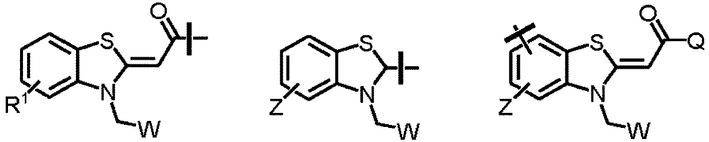 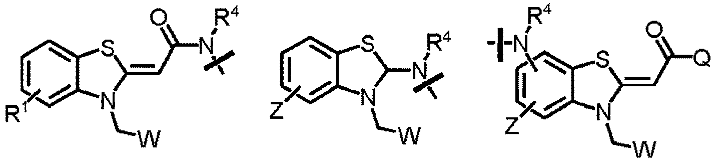
R³ represents a hydrogen atom or an alkyl group, R¹ represents one to four substituents, in a case where R¹ represents two or more substituents, the substituents each independently, and the substituent is a hydrogen atom, an alkyl group, an alkoxy group, a halogen, or a hydroxy group, Y represents a furan group, a thiophene group, a pyrrole group, an imidazole group, a thiazole group, a pyridine group, a pyrazine group, a pyrimidine group, or a triazine group, W represents a hydrogen atom, a halogen, or an alkyl group, Z represents one to three or one to four substituents, in a case where Z represents two or more substituents, the substituents each independently, and the substituent is a hydrogen atom, a halogen, a hydroxy group, an alkyl group, or an alkoxy group, Q represents a halogen, a hydroxy group, an alkyl group, or an alkoxy group, and R⁴ represents a hydrogen atom or an alkyl group.

In one or more embodiments, the compound represented by Formula (IV) is a compound represented by the following formula.

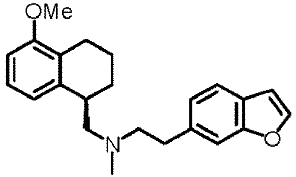

A composition for inhibiting an α2 adrenergic receptor according to the present disclosure, in one or more embodiments, contains a compound represented by Formula (V) below, a prodrug thereof, or a pharmaceutically acceptable salt thereof, as an active ingredient, 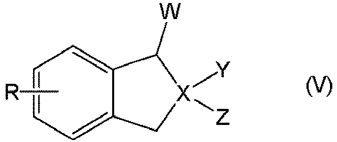
where in Formula (V), R represents one to four substituents, in a case where R represents two or more substituents, the substituents each independently, and the substituent is a hydrogen atom, an alkyl group, an alkoxy group, a halogen, or a hydroxy group, W represents a hydrogen atom, a halogen, or an alkyl group, X represents a nitrogen atom or a carbon atom, and when X represents a carbon atom, Y is present and represents an alkyl group, Z represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following: 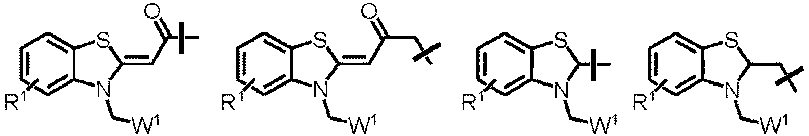 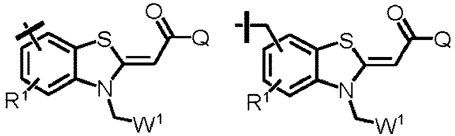
Q represents an alkyl group, an alkoxy group, a halogen, or a hydroxy group, R¹ represents one to three or one to four substituents, in a case where R¹ represents two or more substituents, the substituents each independently, and the substituent is a hydrogen atom, an alkyl group, an alkoxy group, a halogen, or a hydroxy group, and W¹ represents a hydrogen atom, a halogen, or an alkyl group.

In one or more embodiments, the compound represented by Formula (V) is a compound represented by the following formulae.

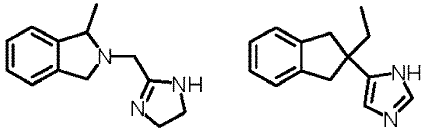

A composition for inhibiting an α2 adrenergic receptor according to the present disclosure, in one or more embodiments, contains a compound represented by Formulae (VI) to (VIII) below, a prodrug thereof, or a pharmaceutically acceptable salt thereof, as an active ingredient, 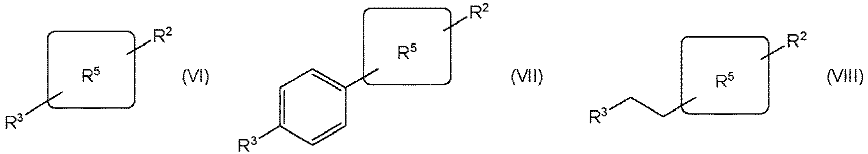 where in Formulae (VI) to (VIII), R² represents an alkyl group or any one of the following: 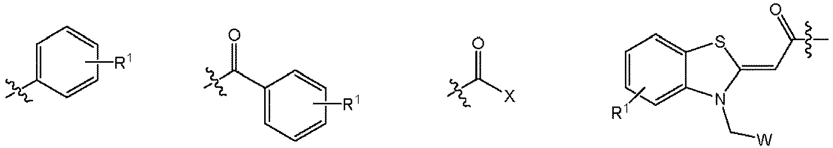 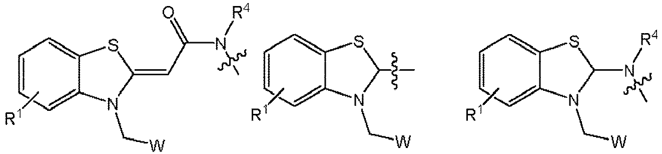 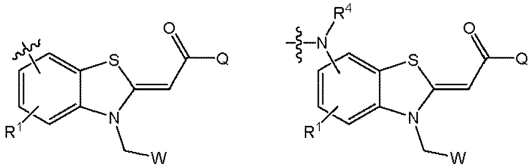
R³ represents any one of the following: 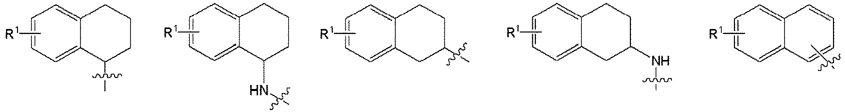 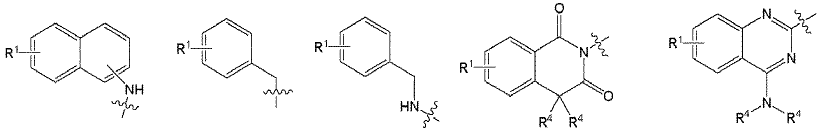 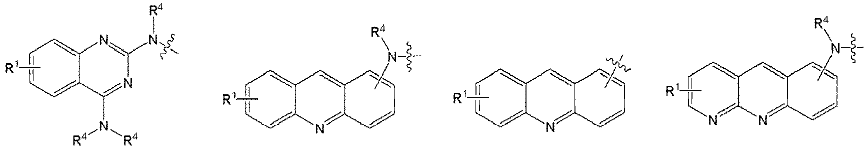 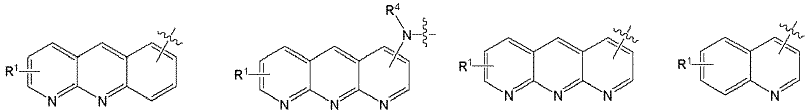 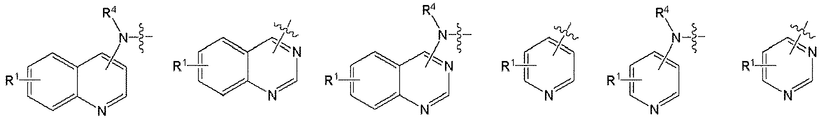 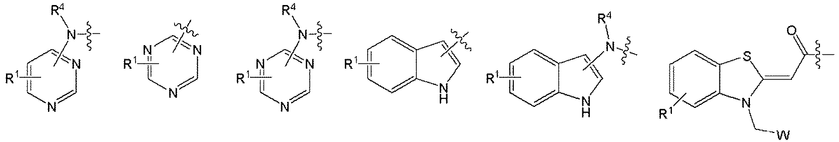 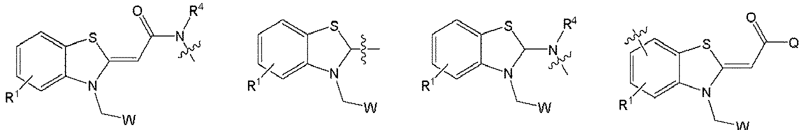 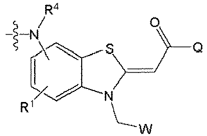
R⁴ represents a hydrogen atom or an alkyl group, W represents a hydrogen atom, a halogen, or an alkyl group, Q represents an alkyl group, an alkoxy group, a halogen, or a hydroxy group, X represents a furan group, a thiophene group, a pyrrole group, an imidazole group, a thiazole group, a pyridine group, a pyrazine group, a pyrimidine group, or a triazine group, W represents a hydrogen atom, a halogen, or an alkyl group, R¹ represents a substituent of an aromatic ring (including a fused ring) and one to seven, one to six, one to five, one to four, one to three, or one or two substituents, and in a case where R¹ represents two or more substituents, the substituents each independently represent a hydrogen atom, an alkyl group, an alkoxy group, or a halogen, and R⁵ represents any one of the following.

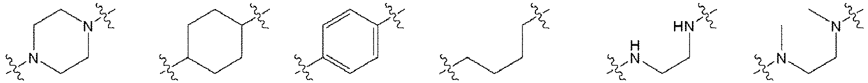

In one or more embodiments, the compound represented by Formula (VI) is a compound represented by Formula (IX) below, 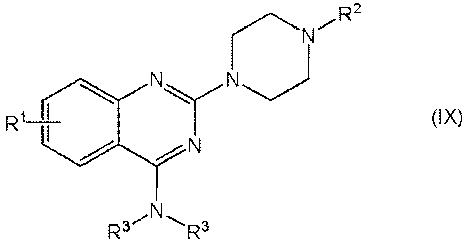 where in Formula (IX), R¹ represents one to four substituents and an alkyl group, an alkoxy group, or a halogen, and R² represents an alkyl group or any one of the following: 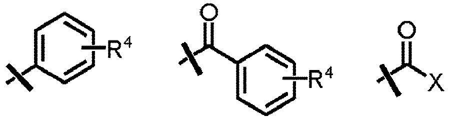 X represents a furan group, a thiophene group, a pyrrole group, an imidazole group, a thiazole group, a pyridine group, a pyrazine group, a pyrimidine group, or a triazine group, R⁴ represents one to four substituents, in a case where R⁴ represents two or more substituents, the substituents each independently, and the substituent is an alkyl group, an alkoxy group, or a halogen, and R³ each independently represent a hydrogen atom or an alkyl group.

In one or more embodiments, the compound represented by Formula (VI) or Formula (IX) is a compound represented by the following formula.

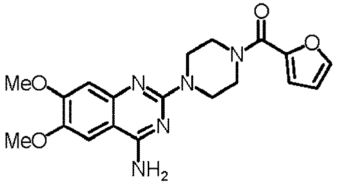

In one or more embodiments, the compound represented by Formula (VII) is a compound represented by Formula (X) below, 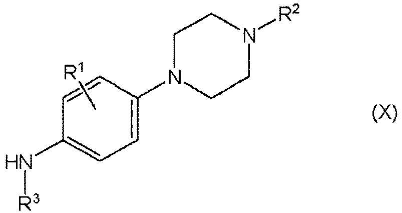 where in Formula (X), R¹ represents an alkyl group, an alkoxy group, or a halogen, and R² represents an alkyl group or any one of the following, 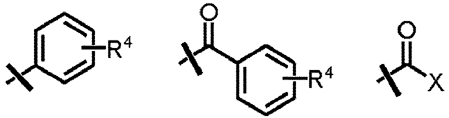 X represents a furan group, a thiophene group, a pyrrole group, an imidazole group, a thiazole group, a pyridine group, a pyrazine group, a pyrimidine group, or a triazine group, R⁴ represents one to four substituents, in a case where R⁴ represents two or more substituents, the substituents each independently, and the substituent is an alkyl group, an alkoxy group, or a halogen, and R³ represents any one of the following.

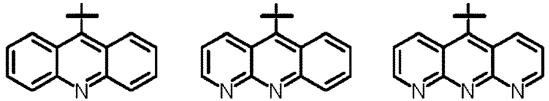

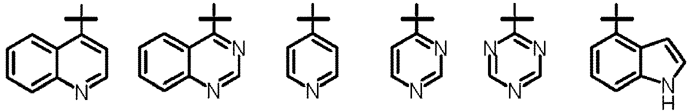

In one or more embodiments, the compound represented by Formula (VII) or Formula (X) is a compound represented by the following formula.

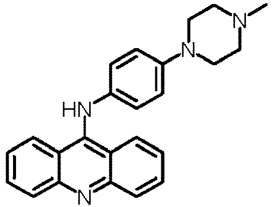

In one or more embodiments, the compound represented by Formula (VIII) is a compound represented by Formula (XI) below, 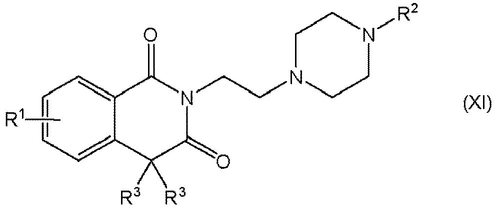 where in Formula (XI), R¹ represents an alkyl group, an alkoxy group, or a halogen, and R² represents an alkyl group or any one of the following: 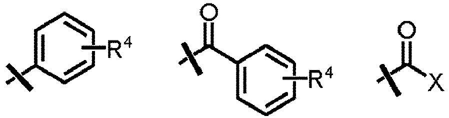 X represents a furan group, a thiophene group, a pyrrole group, an imidazole group, a thiazole group, a pyridine group, a pyrazine group, a pyrimidine group, or a triazine group, R⁴ represents one to four substituents, in a case where R⁴ represents two or more substituents, the substituents each independently, and the substituent is an alkyl group, an alkoxy group, or a halogen, and R³ each independently represent a hydrogen atom or an alkyl group.

In one or more embodiments, examples of the compound represented by Formula (XI) include a compound represented by the formula below.

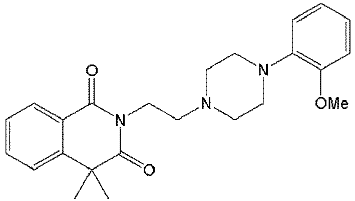

In one or more embodiments, examples of the compound represented by Formula (VIII) include a compound represented by the formula below.

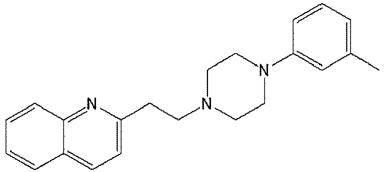

The pharmaceutical composition according to the present disclosure contains the above compounds as active ingredients, and may further contain a pharmaceutically acceptable carrier, an antiseptic agent, a diluent, an excipient, or other pharmaceutically acceptable components.

In the present disclosure, the "pharmaceutically acceptable salt" includes a pharmacologically and/or medically acceptable salt, and examples of the e include an inorganic acid salt, an organic acid salt, an inorganic base salt, an organic base salt, and an acidic or basic amino acid salt.

Preferable examples of the inorganic acid salt include hydrochloride; hydrobromate; sulfate; nitrate; and phosphate. Preferable examples of the organic acid salt include acetate; succinate; fumarate; maleate; tartrate; citrate; lactate; stearate; benzoate; methanesulfonate; and p-toluenesulfonate.

Preferable examples of the inorganic base salt include alkali metal salts such as sodium salt and potassium salt, alkali earth metal salts such as calcium salt and magnesium salt, aluminum salt, and ammonium salt. Preferable examples of the organic base salt include diethylamine salt, diethanolamine salt, meglumine salt, and N,N'-dibenzylethylenediamine salt.

Preferable examples of the acidic amino acid salt include aspartic acid salt, and glutamic acid salt. Preferable examples of the basic amino acid salt include arginine salt, lysine salt, and ornithine salt.

The "salt of the compound" in the present disclosure can include a hydrate formed with the compound when the compound is left to stand in the atmosphere thereby absorbing moisture. Further, in the present disclosure, the "salt of the compound" can also include a solvate formed with the compound when the compound absorbs another certain type of a solvent.

In the present disclosure, examples of the "prodrug", in one or more embodiments, include those which can be easily hydrolyzed in vivo so that the above compound can be reproduced. In a case where the compound is, for example, a compound having a carboxyl group, the prodrug is, for example, the compound in which the carboxyl group has become an alkoxycarbonyl group, the compound in which the carboxyl group has become an alkylthio carbonyl group, or the compound in which the carboxyl group has become an alkylaminocarbonyl group. Further, in a case where the compound is, for example, a compound having an amino group, the prodrug is, for example, the compound in which the amino group is substituted with an alkanoyl group, thereby becoming an alkanoylamino group, the compound in which the amino group is substituted with an alkoxycarbonyl group, thereby becoming an alkoxycarbonyl amino group, the compound in which the amino group has become an acyloxymethylamino group, or the compound in which the amino group has become hydroxylamine. Still further, in a case where the compound is, for example, a compound having a hydroxyl group, the prodrug is, for example, the compound in which the hydroxyl group is substituted with an acyl group, thereby becoming an acyloxy group, the compound in which the hydroxyl group has become a phosphoric acid ester, or the compound in which the hydroxyl group has become an acyloxymethyloxy group. The alkyl part of the group used in the prodrug conversion in these cases is, for example, the alkyl group, and the alkyl group may be substituted (with, for example, an alkoxy group having one to six carbon atoms). In one or more embodiments, in a case of, for example, the compound in which the carboxyl group has become an alkoxycarbonyl group, the alkyl part is a lower alkoxycarbonyl (for example, having one to six carbon atoms) such as methoxycarbonyl or ethoxycarbonyl, or a lower alkoxycarbonyl (for example, having one to six carbon atoms) by substitution with an alkoxy group, such as methoxymethoxycarbonyl, ethoxymethoxycarbonyl, 2-methoxyethoxycarbonyl, 2-methoxyethoxymethoxycarbonyl, or pivaloyloxymethoxycarbonyl.

The "pharmaceutical composition" according to the present disclosure, in one or more embodiments, can be in a dosage form suitable for administration, obtained by applying a well-known formulation technology. The administration form is, for example, oral administration in a dosage form such as a tablet, a capsule, a granule, a powder, a pill, a troche, a syrup, or a liquid, though the dosage form is not limited to these. Alternatively, the administration form is, for example, non-oral administration in a dosage form such as an injection, a liquid, an aerosol, a suppository, a plaster, a poultice, a lotion, a liniment, an ointment, or an eye drop. These formulations can be produced by a well-known method using an additive such as an excipient, a coating agent, a binder, a disintegrator, a stabilizer, a flavoring agent, and a diluent, though the additive is not limited to these.

Examples of the excipient include: starches such as starch, potato starch, and corn starch; lactose; crystalline cellulose; and phosphoric acid hydrogen calcium, though the excipient is not limited to these. Examples of the coating agent include ethyl cellulose, hydroxypropyl cellulose, hydroxypropyl methyl cellulose, shellac, talc, carnauba wax, and paraffin, though the coating agent is not limited to these. Examples of the binder include polyvinylpyrrolidone, macrogol, and the same compounds as those mentioned as the examples of the excipient, though the binder is not limited to these. Examples of the disintegrator include the same compounds as those mentioned as the examples of the excipient, as well as chemically modified starches and celluloses such as croscarmellose sodium, carboxymethyl starch sodium, and cross-linked polyvinylpyrrolidone, though the disintegrator is not limited to these. Examples of the stabilizer include: paraoxybenzoic acid esters such as methylparaben and propylparaben; alcohols such as chlorobutanol, benzyl alcohol, phenyl ethyl alcohol; benzalkonium chloride; phenols such as phenol and cresol; thimerosal; dehydroacetic acid; and sorbic acid, though the stabilizer is not limited to these. Examples of the flavoring agent include sweeteners, acidifiers, and aromas that are typically used, though the flavoring agent is not limited to these.

Further, in the production of a liquid agent, ethanol, phenol, chlorocresol, purification water, distilled water or the like can be used as a solvent, though the solvent is not limited to these. Besides, a surfactant, an emulsifier, and the like also can be used as required. Examples of the foregoing surfactant and emulsifier include polysorbate 80, polyoxyl stearate 40, and lauromacrogol, though the surfactant and the emulsifier are not limited to these.

The "pharmaceutical composition" according to the present disclosure, in one or more embodiments, is to be administered to a target who has pain.

Examples of the target include humans and animals other than humans. In one or more embodiments, examples of the animals include mammals such as mice, rats, guinea pigs, hamsters, rabbits, cats, dogs, sheep, pigs, cows, horses, goats, and monkeys.

A dose of the pharmaceutical composition according to the present disclosure can vary with the symptom, the age, the administering method, and the like. The method for using the pharmaceutical composition according to the present disclosure can be a method of intermittently or continuously administering the compound serving as an active ingredient, orally, percutaneously, submucosally, subcutaneously, intramuscularly, intravascularly, intracerebrally, or intraperitoneally in such a manner that the concentration in the body is in a range of 100 nM to 1 mM, though the method is not limited to these.

For example, in a non-limited embodiment, in a case of oral administration, it can be considered to administer depending on the symptom in one time or in several times to a target (an adult if the target is human) in a daily amount, calculated as a free compound of active ingredient, of from 0.01 mg (preferably 0.1 mg) as a lower limit to 2000 mg (preferably 500 mg, more preferably 100 mg) as an upper limit. In a non-limited embodiment, for example, in a case of intravenous administration, the method is such a method of administration to a target (an adult if the target is a human) in an amount from a lower limit of 0.001 mg (preferably 0.01 mg), to an upper limit of 500 mg (preferably 50 mg) per one day, in one dose, or divided into several doses, according to the symptom.

The present disclosure, in another aspect, relates to a method for providing analgesia through inhibition of an α2 adrenergic receptor, the method including administering an effective dose of the pharmaceutical composition according to the present disclosure to a target.

The present disclosure, in one or more embodiments, relates to a method for providing analgesia through inhibition of an α2 adrenergic receptor by administering at least one selected from the group consisting of a compound represented by Formulae (I) to (VIII) above, a prodrug thereof, a pharmaceutically acceptable salt thereof, and a combination thereof.

The present disclosure, in one or more embodiments, relates to use of at least one selected from the group consisting of a compound represented by Formulae (I) to (VIII) above, a prodrug thereof, a pharmaceutically acceptable salt thereof, and a combination thereof, for providing analgesia through inhibition of an α2 adrenergic receptor.

### [Method for inhibiting α2 adrenergic receptors and use]

The present disclosure, in one or more embodiments, relates to a method for inhibiting an α2 adrenergic receptor by administering at least one selected from the group consisting of a compound represented by Formulae (I) to (VIII) above, a prodrug thereof, a pharmaceutically acceptable salt thereof, and a combination thereof.

The present disclosure, in one or more embodiments, relates to use of at least one selected from the group consisting of a compound represented by Formulae (I) to (VIII) above, a prodrug thereof, a pharmaceutically acceptable salt thereof, and a combination thereof, for inhibiting an α2 adrenergic receptor.

The present disclosure can relate to one or more embodiments described below:
[1] A pharmaceutical composition for analgesia, comprising an alpha-2 adrenergic receptor antagonist as an active ingredient.
[2] The pharmaceutical composition according to [1], comprising an alpha-2B adrenergic receptor antagonist as an active ingredient.
[3] A composition for inhibiting an alpha-2 adrenergic receptor, comprising a compound represented by Formula (I) below, a prodrug thereof, or a pharmaceutically acceptable salt thereof, 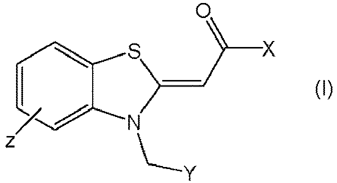 where in Formula (I), X represents an alkyl group, an alkoxy group, a halogen, a hydroxy group, an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, Y represents a hydrogen atom, a halogen, or an alkyl group, and Z represents one to four substituents, in a case where Z represents two or more substituents, the substituents each independently, and the substituent is a hydrogen atom, an alkyl group, an alkoxy group, a halogen, or a hydroxy group.
[4] A composition for inhibiting an alpha-2 adrenergic receptor, comprising a compound represented by Formula (II) below, a prodrug thereof, or a pharmaceutically acceptable salt thereof as an active ingredient, 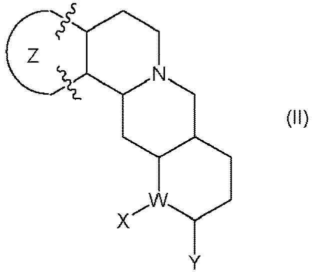 where in Formula (II), W represents CH or a nitrogen atom, X represents an alkoxycarbonyl group, a carboxyl group, an alkylsulfonyl group, an alkylsulfinyl group, a sulfonyl group, a sulfinyl group, a sulfonic acid alkyl ester group, a sulfinic acid alkyl ester group, or a hydrogen atom, Y represents a hydroxyl group, an alkoxy group, a halogen, or a hydrogen atom, and Z represents a fused ring or any one of the following: 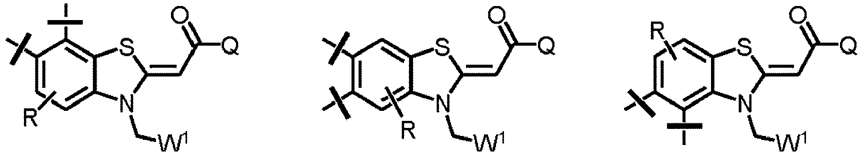 Q represents an alkyl group, an alkoxy group, a halogen, or a hydroxy group, R represents one or two substituents, and in a case where R represents two substituents, the two substituents each independently represent a hydrogen atom, an alkyl group, an alkoxy group, a halogen, or a hydroxy group, and W¹ represents a hydrogen atom, a halogen, or an alkyl group.
[5] A composition for inhibiting an alpha-2 adrenergic receptor, comprising a compound represented by Formula (III) below, a prodrug thereof, or a pharmaceutically acceptable salt thereof as an active ingredient, 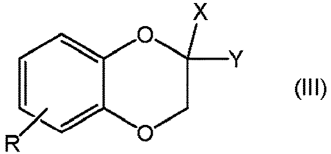 where in Formula (III), R represents one to four substituents, in a case where R represents two or more substituents, the substituents each independently, and the substituent is a hydrogen atom, an alkyl group, an alkoxy group, a halogen, or a hydroxy group, X represents an alkyl group, an alkoxy group, or a hydrogen atom, and Y represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following: 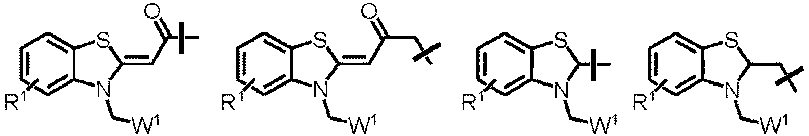 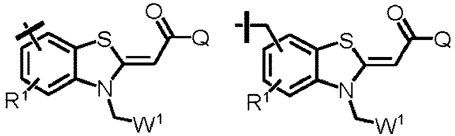 Q represents an alkyl group, an alkoxy group, a halogen, or a hydroxy group, R¹ represents one to three or one to four substituents, in a case where R¹ represents two or more substituents, the substituents each independently, and the substituent is a hydrogen atom, an alkyl group, an alkoxy group, a halogen, or a hydroxy group, and W¹ represents a hydrogen atom, a halogen, or an alkyl group.
[6] A composition for inhibiting an alpha-2 adrenergic receptor, comprising a compound represented by Formula (IV) below, a prodrug thereof, or a pharmaceutically acceptable salt thereof as an active ingredient, 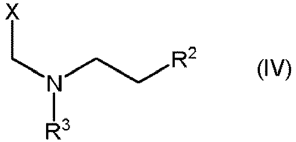
   where in Formula (IV), X represents any one of the following, 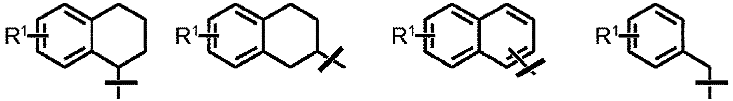
   R² represents an indole group, a quinoline group, or a quinazoline group, or any one of the following: 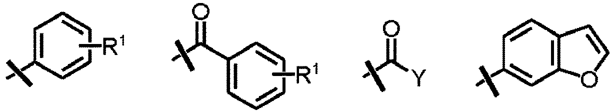 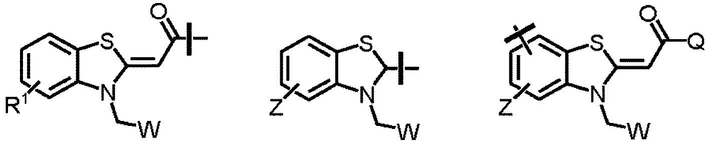 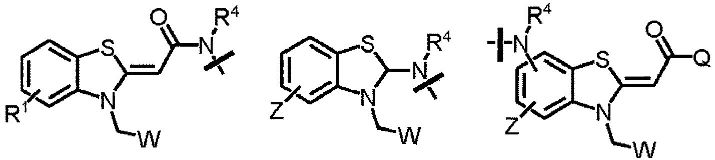
   R³ represents a hydrogen atom or an alkyl group, R¹ represents one to four substituents, in a case where R¹ represents two or more substituents, the substituents each independently, and the substituent is a hydrogen atom, an alkyl group, an alkoxy group, a halogen, or a hydroxy group, Y represents a furan group, a thiophene group, a pyrrole group, an imidazole group, a thiazole group, a pyridine group, a pyrazine group, a pyrimidine group, or a triazine group, W represents a hydrogen atom, a halogen, or an alkyl group, Z represents one to three or one to four substituents, in a case where Z represents two or more substituents, the substituents each independently, and the substituent is a hydrogen atom, a halogen, a hydroxy group, an alkyl group, or an alkoxy group, Q represents a halogen, a hydroxy group, an alkyl group, or an alkoxy group, and R⁴ represents a hydrogen atom or an alkyl group.
[7] A composition for inhibiting an alpha-2 adrenergic receptor, comprising a compound represented by Formula (V) below, a prodrug thereof, or a pharmaceutically acceptable salt thereof as an active ingredient, 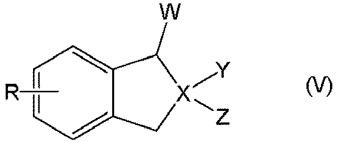 where in Formula (V), R represents one to four substituents, in a case where R represents two or more substituents, the substituents each independently, and the substituent is a hydrogen atom, an alkyl group, an alkoxy group, a halogen, or a hydroxy group, W represents a hydrogen atom, a halogen, or an alkyl group, X represents a nitrogen atom or a carbon atom, and when X represents a carbon atom, Y is present and represents an alkyl group, Z represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following: 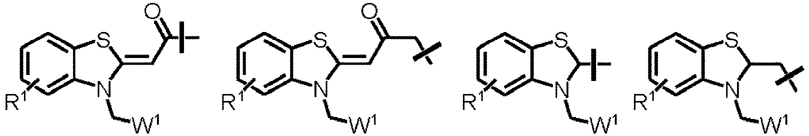 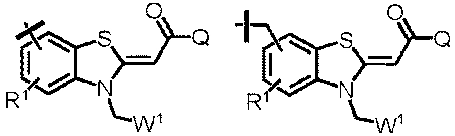 Q represents an alkyl group, an alkoxy group, a halogen, or a hydroxy group, R¹ represents one to three or one to four substituents, in a case where R¹ represents two or more substituents, the substituents each independently, and the substituent is a hydrogen atom, an alkyl group, an alkoxy group, a halogen, or a hydroxy group, and W¹ represents a hydrogen atom, a halogen, or an alkyl group.
[8] A composition for inhibiting an alpha-2 adrenergic receptor, comprising a compound represented by Formulae (VI) to (VIII) below, a prodrug thereof, or a pharmaceutically acceptable salt thereof as an active ingredient, 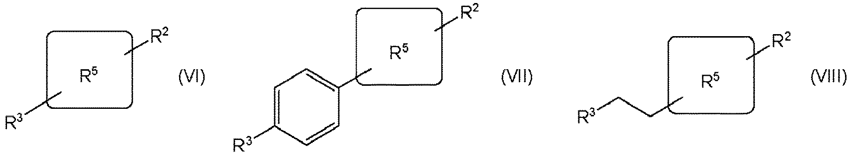
   where in Formulae (VI) to (VIII), R² represents an alkyl group or any one of the following, 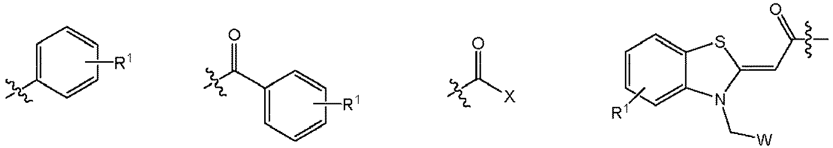 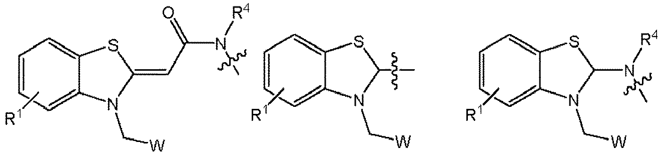 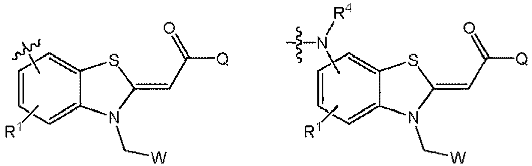
   R³ represents any one of the following: 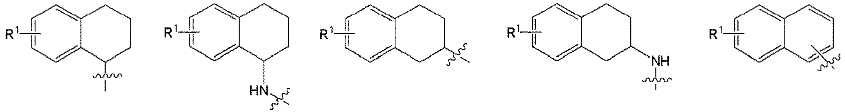 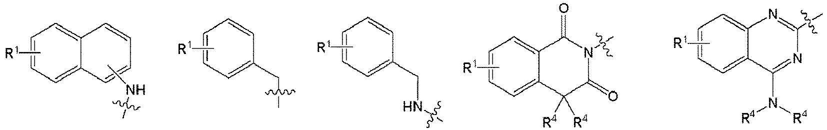 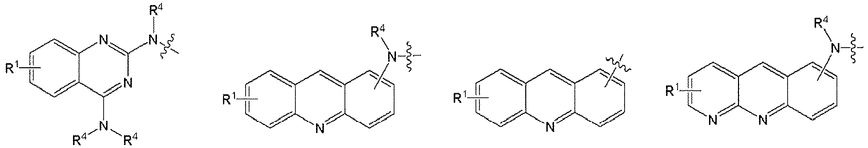 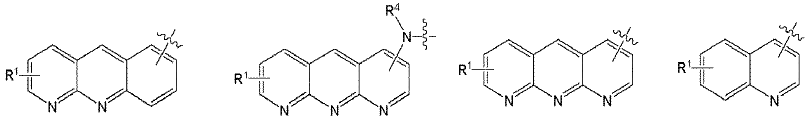 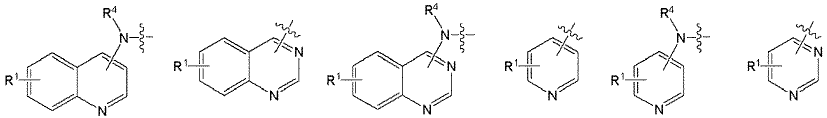 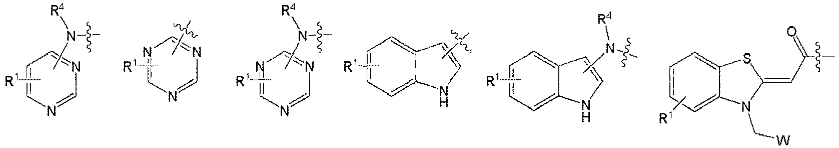 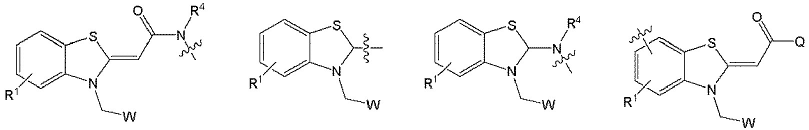 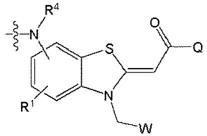
   R⁴ represents a hydrogen atom or an alkyl group, W represents a hydrogen atom, a halogen, or an alkyl group, Q represents an alkyl group, an alkoxy group, a halogen, or a hydroxy group, X represents a furan group, a thiophene group, a pyrrole group, an imidazole group, a thiazole group, a pyridine group, a pyrazine group, a pyrimidine group, or a triazine group, W represents a hydrogen atom, a halogen, or an alkyl group, R¹ represents one to seven, one to six, one to five, one to four, one to three, or one or two substituents, in a case where R¹ represents two or more substituents, the substituents each independently, and the substituent is a hydrogen atom, an alkyl group, an alkoxy group, or a halogen, and R⁵ represents any one of the following: 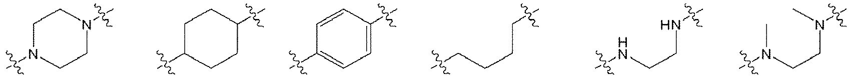
[9] A pharmaceutical composition for analgesia through inhibition of an alpha-2 adrenergic receptor, comprising, as an active ingredient, at least one selected from the group consisting of the compound represented by Formulae (I) to (VIII) according to any one of [3] to [8], the prodrug thereof, the pharmaceutically acceptable salt thereof, and a combination thereof.
[10] A method for inhibiting an alpha-2 adrenergic receptor, including administering at least one selected from the group consisting of the compound represented by Formulae (I) to (VIII) according to any one of [3] to [8], the prodrug thereof, the pharmaceutically acceptable salt thereof, and a combination thereof.
[11] A method for relieving pain through inhibition of an alpha-2 adrenergic receptor, including administering at least one selected from the group consisting of the compound represented by Formulae (I) to (VIII) according to any one of [3] to [8], the prodrug thereof, the pharmaceutically acceptable salt thereof, and a combination thereof.
[12] Use of at least one selected from the group consisting of the compound represented by Formulae (I) to (VIII) according to any one of [3] to [8], the prodrug thereof, the pharmaceutically acceptable salt thereof, and a combination thereof, for inhibiting an alpha-2 adrenergic receptor.
[13] Use of at least one selected from the group consisting of the compound represented by Formulae (I) to (VIII) according to any one of [3] to [8], the prodrug thereof, the pharmaceutically acceptable salt thereof, and a combination thereof, for relieving pain through inhibition of an alpha-2 adrenergic receptor.

### Example

The following description describes the present disclosure in more details by way of examples. These examples however are merely illustrative, and the present disclosure is not limited to these examples. The all of the documents cited in the present disclosure are incorporated in the present disclosure.

### Production example 1: Production of compound 1

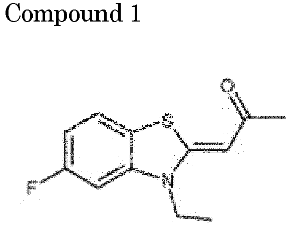

The compound 1 was synthesized in the following manner.

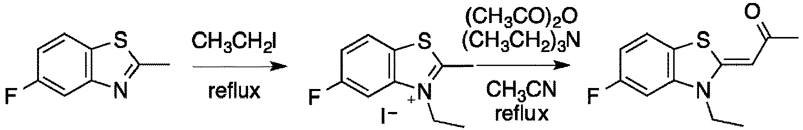

Ethyl iodide (iodoethane) (50.0 mL, 622 mmol, commercial product) solution of 5-fluoro-2-methylbenzothiazole (48.8 g, 292 mmol, commercial product) was heated and refluxed (oil bath temperature 100 °C) for 72 hours. After the solution was cooled at room temperature, a colorless solid substance formed was collected by filtration, washed with ethyl acetate, and dried under a reduced pressure, whereby 3-ethyl-5-fluoro-2-methylbenzothiazolium iodide (46.5 g, 151 mmol, 51.7 %) was obtained in a colorless solid state.

Next, in an argon atmosphere, acetic anhydride (22.5 mL, 236 mmol, commercial product) and triethylamine (32.2 mL, 231 mmol, commercial product) were added sequentially to an acetonitrile solution (150 mL) of 3-ethyl-5-fluoro-2-methylbenzothiazolium iodide (32.3 g, 100 mmol) at room temperature, and thereafter, this was heated and refluxed (oil bath temperature 80 °C) for three hours. After the reaction mixture thus obtained was cooled at room temperature, water was added thereto, and the reaction mixture was extracted with ethyl acetate (×3). This was washed with a saturated saline solution, and thereafter, was dried with anhydrous sodium sulfate. After this was filtered, a filtrate thus obtained was concentrated under a reduced pressure. The obtained crude reaction product was purified by silica gel column chromatography (Wako Pure Chemical Industries, Ltd., Presep (trademark) Silica Gel (HC-N) Type2L, hexane/ethyl acetate = 5/1 to 1/1), and thereafter, recrystallized with hexane-ethyl acetate. Thus, (1Z)-1-(3-ethyl-5-fluoro-2(3H)-benzothiazolylidene)-2-propanone (17.8 g, 75.2 mmol, 75.2 %) (Compound 1) was obtained in a colorless needle crystal form. Melting point, 179-180 °C; TLC Rf 0.36 (hexane/ethyl acetate = 7/3); ¹H NMR (CDCl₃, 400 MHz) δ 1.38 (t, 3H, J = 7.2 Hz), 2.25 (s, 3H),4.01 (q, 2H, J = 7.2 Hz), 5.90 (s, 1 H), 6.82 (dd, 1H, J = 2.4, 9.6Hz), 6.88 (ddd, 1H, J = 2.4, 8.4, 8.4 Hz), 7.48 (dd, 1H, J = 5.2, 8.4 Hz); IR (KBr, cm⁻¹) 978, 1042, 1125, 1192, 1326, 1332, 1354, 1382, 1449, 1453, 1468, 1488, 1492, 1604, 2981.

### Production example 2: Production of compound 2

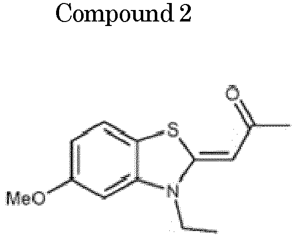

The compound 2 was synthesized according to the technique disclosed in the following document (M. Muraki, et al., Manipulation of Alternative Splicing by a Newly Developed Inhibitor of Clks, The Journal of Biological Chemistry, 2004, 279, 24246-24254, or WO2010/010797 A1).

### Production example 3: Production of compound 3

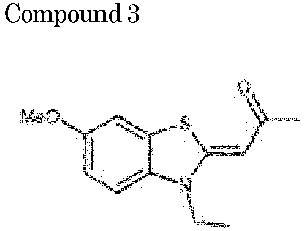

The compound 3 was synthesized in the following manner.

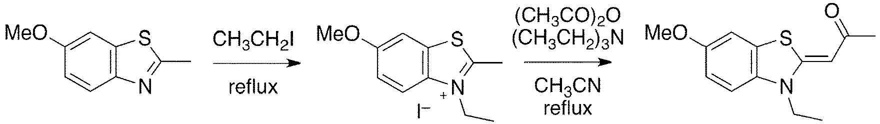

Ethyl iodide (iodoethane) (2.00 mL, 24.6 mmol, commercial product) solution of 6-methoxy-2-methylbenzothiazole (201 mg, 1.12 mmol, commercial product) was heated and refluxed (oil bath temperature 100 °C) for 24 hours. After the solution was cooled at room temperature, a colorless solid substance formed was collected by filtration, washed with ethyl acetate, and dried under a reduced pressure, whereby 3-ethyl-6-methoxy-2-methylbenzothiazolium iodide (257 mg, 0.766 mmol, 68.4 %) was obtained in a colorless solid state.

In an argon atmosphere, acetic anhydride (0.170 mL, 1.80 mmol, commercial product), and triethylamine (0.250 mL, 1.79 mmol, commercial product) were added sequentially to an acetonitrile solution (2 mL) of 3-ethyl-6-methoxy-2-methylbenzothiazolium iodide (257 mg, 0.766 mmol) at room temperature, and thereafter, this was heated and refluxed (oil bath temperature 80 °C) for two hours. After the reaction mixture thus obtained was cooled at room temperature, this reaction mixture was concentrated under a reduced pressure, water was added thereto, and the reaction mixture was extracted with ethyl acetate (×3). This was washed with a saturated saline solution, and thereafter, was dried with anhydrous sodium sulfate. After this was filtrated, a filtrate thus obtained was concentrated under a reduced pressure. The obtained crude reaction product was purified by silica gel column chromatography (hexane/ethyl acetate =1/2), and (1Z)-1-(3-ethyl-6-methoxy-2(3H)-benzothiazoylidene)-2-propanone (115 mg, 0.461 mmol, 60.2 %) (Compound 3) was obtained in a light yellow solid state. Melting point 134-135 °C; TLC Rf 0.27 (hexane/ethyl acetate = 1/2); ¹H NMR (CDCl₃, 400 MHz) δ 1.36 (t, 3H, J = 7.2 Hz), 2.23 (s, 3H), 4.03 (q, 2H, J = 7.2 Hz), 5.82 (s, 1 H), 6.91 (dd, 1H, J = 2.5, 8.7 Hz), 7.01 (d,1H, J = 8.7 Hz), 7.13 (d, 1H, J = 2.5 Hz); IR (KBr, cm⁻¹) 720, 760, 801, 959, 1020, 1046, 1136, 1188, 1219, 1258, 1273, 1298, 1327, 1358, 1472, 1487, 1590, 1603, 2342, 2361, 2980.

### [Binding affinity to α1 adrenergic receptors]

The compound 1 was assayed for its inhibitory activity against α1A adrenergic receptor and α1B adrenergic receptor using prazosin with a 7-methoxy group labeled with tritium as a tracer, and binding affinity was examined. Prazosin is an antagonist that specifically binds to α1 adrenergic receptors.

As a result, the compound 1 did not exhibit binding affinity to the α1A adrenergic receptor or the α1B adrenergic receptor.

### [Binding affinity to α2 adrenergic receptors]

The compound 1 was assayed for its inhibitory activity againsta2A adrenergic receptor and α2B adrenergic receptor using rauwolscine with a methyl group labeled with tritium as a tracer, and binding affinity was examined. Rauwolscine is an antagonist that specifically binds to α2 adrenergic receptors.

As a result, the compound 1 exhibited selective binding affinity to α2A adrenergic receptor and α2B adrenergic receptor. The binding affinity of the compound 1 to α2B adrenergic receptor was higher than to α2A adrenergic receptor.

### [TGFα shedding assay]

The degree of inhibiting signal transduction of α2A adrenergic receptor, α2B adrenergic receptor, and α2C adrenergic receptor in response to noradrenaline stimulation, using compounds 1 to 3 was checked through a TGFα shedding assay. The TGFα shedding assay was performed according to the method disclosed in WO2015/128894A1.

Specifically, human embryonic kidney HEK293 cells were transfected with a plasmid vector containing the cDNA of alkaline phosphatase (AP) fused TGFα, a plasmid vector containing the cDNA of a fusion protein (Gaq/i) of an N-terminal peptide fragment of Gα protein Gaq and a C-terminal peptide fragment of Go protein Gai, and a plasmid vector containing the cDNA of a target GPCR, 24 hours later a compound was added and cultured for 30 minutes, a GPCR agonist (5 nM noradrenaline) was added, one hour later half of the culture supernatant was transferred to a new plate to prepare a supernatant plate, and the remaining plate was used as a cell plate. Ap-nitrophenyl phosphate solution was added to each plate and allowed to stand, and when 0 hours and 2 hours had passed, the absorbance at 405 nm was measured using a plate reader.

The measured value was converted to AP activity (%) according to the method described in WO2015/128894A1, and the obtained value was used as %Shedding.

The results are illustrated in FIG. 1 and Table 1.

**[Table 1]**

| IC50 (µM), shedding assay (5 nM Noradrenaline stimulation) | | | |
|---|---|---|---|
| Compound | α2A | α2B | α2C |
| Compound 2 | > 30 | > 30 | > 30 |
| Compound 1 | > 30 | 1.2 | > 30 |
| Compound 3 | > 30 | > 30 | > 30 |

As illustrated in FIG. 1, the compounds 1 to 3 can function as α2 adrenergic receptor antagonists. Further, as illustrated in Table 1, the compound 1 had higher binding affinity (inhibitory activity) to α2B adrenergic receptor than to α2A adrenergic receptor and α2C adrenergic receptor.

### [Pain reduction]

Pain reduction effects of the compounds 1 to 3 were checked using pain model mice.
Compound: compounds 1 and 2
Pain model mouse: inflammatory pain model mice induced by carrageenan

After subcutaneous plantar administration of carrageenan, acute inflammation develops over several hours, which causes a phenomenon of hypersensitivity to mechanical stimulation and/or thermal stimulation. A therapeutic agent containing the compound 1 or 2 as an active ingredient was administered to each of model mice with this carrageenan-induced inflammatory pain, and pain was evaluated.

At six hours after carrageenan was subcutaneously administered to a hind limb sole of each mouse (male, C57BL/6J, 6 to 8 weeks old), the compound 1 or 2 was administered intrathecally, and at one hour after the administration of the compound 1 or 2, therapeutic effects thereof with respect to mechanical allodynia were determined.

### Compound: compound 3

### Pain model mouse: inflammatory pain model mouse induced by complete Freund's adjuvant (CFA)

Subcutaneous plantar administration of CFA causes inflammation due to the initiation of immune response, which causes a phenomenon of hypersensitivity to mechanical stimulation and/or thermal stimulation at three days after the administration. A therapeutic agent containing the compound 3 as an active ingredient was administered to each of model mice with this CFA-induced inflammatory pain, and pain was evaluated.

At three days after CFA was subcutaneously administered to a hind limb sole of each mouse (male, C57BL/6J, 6 to 8 weeks old), the compound 3 was administered intrathecally, and therapeutic effects thereof with respect to mechanical allodynia were determined up to three hours after the administration of the compound 3.

Results: The compounds 1 to 3 significantly reduced pain in inflammatory pain model mice induced by carrageenan or CFA.

Therefore, it is confirmed that the compounds 1 to 3 function as α2 adrenergic receptor antagonists, and provide analgesia through their antagonism.

## Claims

1. A pharmaceutical composition for analgesia, comprising an alpha-2 adrenergic receptor antagonist as an active ingredient.

2. The pharmaceutical composition according to claim 1, comprising an alpha-2B adrenergic receptor antagonist as an active ingredient.

3. A composition for inhibiting an alpha-2 adrenergic receptor, comprising
a compound represented by the following Formula (I), a prodrug thereof, or a pharmaceutically acceptable salt thereof, 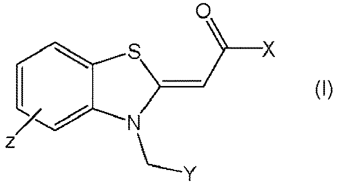 where in Formula (I),
X represents an alkyl group, an alkoxy group, a halogen, a hydroxy group, an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group,
Y represents a hydrogen atom, a halogen, or an alkyl group, and
Z represents one to four substituents, in a case where Z represents two or more substituents, the substituents each independently, and the substituent is a hydrogen atom, an alkyl group, an alkoxy group, a halogen, or a hydroxy group.

4. A composition for inhibiting an alpha-2 adrenergic receptor, comprising
a compound represented by the following Formula (II), a prodrug thereof, or a pharmaceutically acceptable salt thereof as an active ingredient, 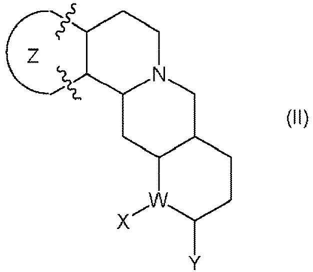 where in Formula (II),
W represents CH or a nitrogen atom,
X represents an alkoxycarbonyl group, a carboxyl group, an alkylsulfonyl group, an alkylsulfinyl group, a sulfonyl group, a sulfinyl group, a sulfonic acid alkyl ester group, a sulfinic acid alkyl ester group, or a hydrogen atom,
Y represents a hydroxyl group, an alkoxy group, a halogen, or a hydrogen atom, and
Z represents a fused ring or any one of the following, 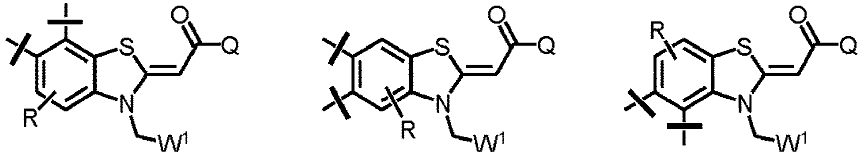
where
Q represents an alkyl group, an alkoxy group, a halogen, or a hydroxy group,
R represents one or two substituents, and in a case where R represents two substituents, the two substituents each independently represent a hydrogen atom, an alkyl group, an alkoxy group, a halogen, or a hydroxy group, and
W¹ represents a hydrogen atom, a halogen, or an alkyl group.

5. A composition for inhibiting an alpha-2 adrenergic receptor, comprising,
a compound represented by the following Formula (III), a prodrug thereof, or a pharmaceutically acceptable salt thereof as an active ingredient, 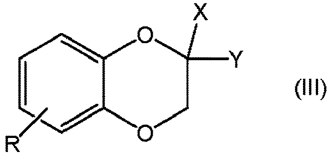 where in Formula (III),
R represents one to four substituents, in a case where R represents two or more substituents, the substituents each independently, and the substituent is a hydrogen atom, an alkyl group, an alkoxy group, a halogen, or a hydroxy group,
X represents an alkyl group, an alkoxy group, or a hydrogen atom, and
Y represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following, 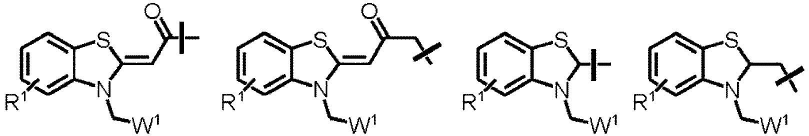 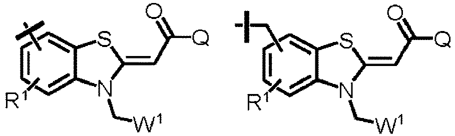
where
Q represents an alkyl group, an alkoxy group, a halogen, or a hydroxy group,
R¹ represents one to three or one to four substituents, in a case where R¹ represents two or more substituents, the substituents each independently, and the substituent is a hydrogen atom, an alkyl group, an alkoxy group, a halogen, or a hydroxy group, and
W¹ represents a hydrogen atom, a halogen, or an alkyl group.

6. A composition for inhibiting an alpha-2 adrenergic receptor, comprising
a compound represented by the following Formula (IV), a prodrug thereof, or a pharmaceutically acceptable salt thereof as an active ingredient, 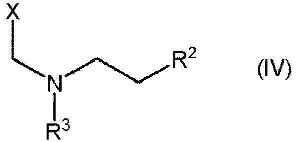 where in Formula (IV),
X represents any one of the following, 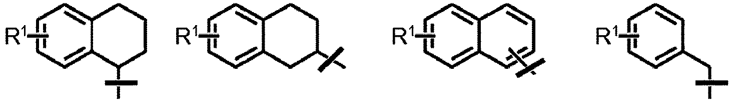
R² represents an indole group, a quinoline group, or a quinazoline group, or any one of the following, 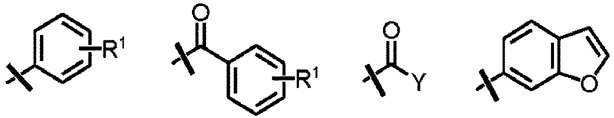 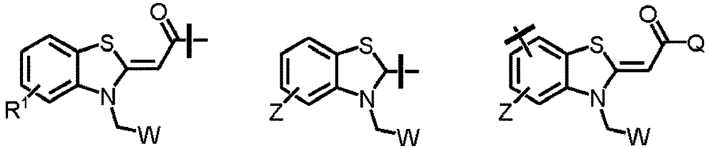 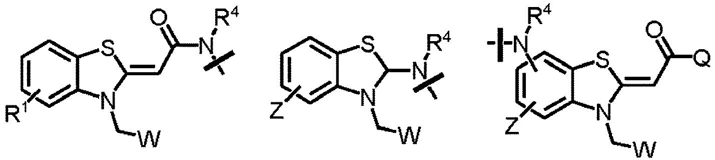
R³ represents a hydrogen atom or an alkyl group,
where
R¹ represents one to four substituents, in a case where R¹ represents two or more substituents, the substituents each independently, and the substituent is a hydrogen atom, an alkyl group, an alkoxy group, a halogen, or a hydroxy group,
Y represents a furan group, a thiophene group, a pyrrole group, an imidazole group, a thiazole group, a pyridine group, a pyrazine group, a pyrimidine group, or a triazine group,
W represents a hydrogen atom, a halogen, or an alkyl group,
Z represents one to three or one to four substituents, in a case where Z represents two or more substituents, the substituents each independently, and the substituent is a hydrogen atom, a halogen, a hydroxy group, an alkyl group, or an alkoxy group,
Q represents a halogen, a hydroxy group, an alkyl group, or an alkoxy group, and
R⁴ represents a hydrogen atom or an alkyl group.

7. A composition for inhibiting an alpha-2 adrenergic receptor, comprising
a compound represented by the following Formula (V), a prodrug thereof, or a pharmaceutically acceptable salt thereof as an active ingredient, 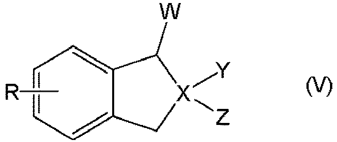 where in Formula (V),
R represents one to four substituents, in a case where R represents two or more substituents, the substituents each independently, and the substituent is a hydrogen atom, an alkyl group, an alkoxy group, a halogen, or a hydroxy group,
W represents a hydrogen atom, a halogen, or an alkyl group,
X represents a nitrogen atom or a carbon atom,
when X represents a carbon atom, Y is present and represents an alkyl group,
Z represents an imidazoyl group, an imidazoylmethyl group, an N-alkyl imidazoyl group, an N-alkyl imidazoylmethyl group, a 4,5-dihydroimidazoyl group, a 4,5-dihydroimidazoylmethyl group, an N-alkyl-4,5-dihydroimidazoyl group, an N-alkyl-4,5-dihydroimidazoylmethyl group, a pyridyl group, a pyridylmethyl group, a pyrazyl group, a pyrazylmethyl group, a pyrimidyl group, a pyrimidylmethyl group, a triazinyl group, a triazinylmethyl group, a thiazolyl group, a thiazolylmethyl group, a thiadiazolyl group, or a thiadiazolylmethyl group, or any one of the following, 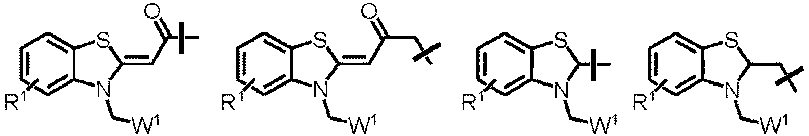 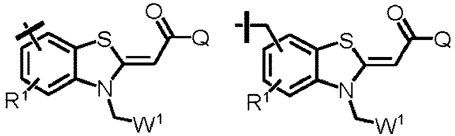
where
Q represents an alkyl group, an alkoxy group, a halogen, or a hydroxy group,
R¹ represents one to three or one to four substituents, in a case where R¹ represents two or more substituents, the substituents each independently, and the substituent is a hydrogen atom, an alkyl group, an alkoxy group, a halogen, or a hydroxy group, and
W¹ represents a hydrogen atom, a halogen, or an alkyl group.

8. A composition for inhibiting an alpha-2 adrenergic receptor, comprising
a compound represented by the following Formulae (VI) to (VIII), a prodrug thereof, or a pharmaceutically acceptable salt thereof as an active ingredient, 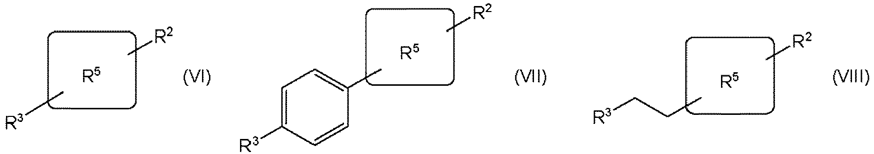 where in Formulae (VI) to (VIII),
R² represents an alkyl group or any one of the following, 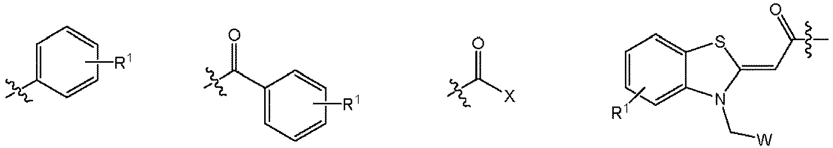 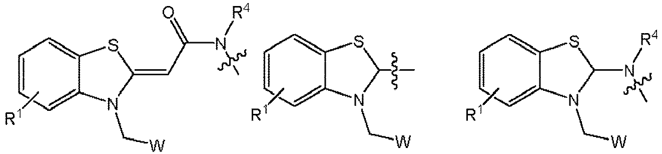 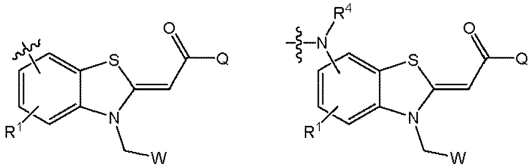
R³ represents any one of the following, 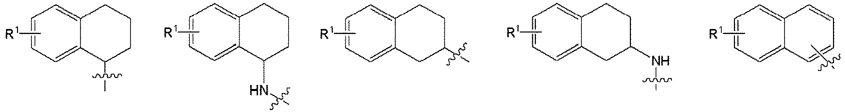 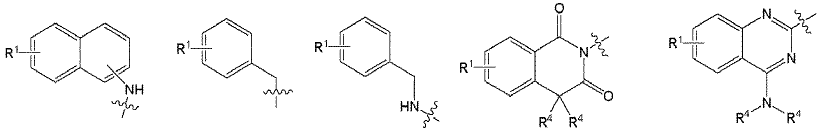 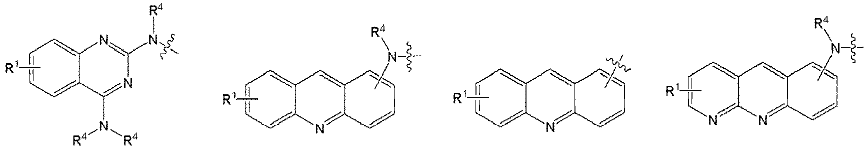 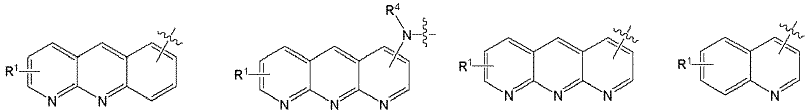 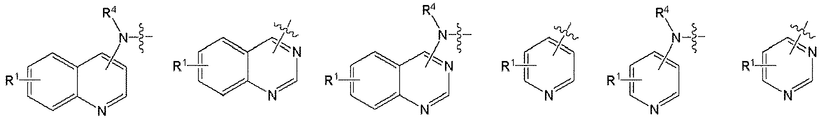 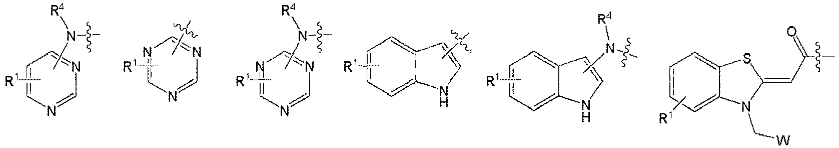 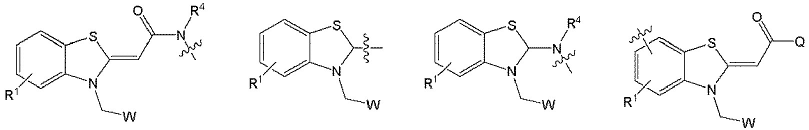 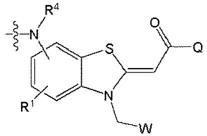
where
R⁴ represents a hydrogen atom or an alkyl group,
W represents a hydrogen atom, a halogen, or an alkyl group,
Q represents an alkyl group, an alkoxy group, a halogen, or a hydroxy group,
X represents a furan group, a thiophene group, a pyrrole group, an imidazole group, a thiazole group, a pyridine group, a pyrazine group, a pyrimidine group, or a triazine group,
W represents a hydrogen atom, a halogen, or an alkyl group, and
R¹ represents one to seven, one to six, one to five, one to four, one to three, or one or two substituents, in a case where R¹ represents two or more substituents, the substituents each independently, and the substituent is a hydrogen atom, an alkyl group, an alkoxy group, or a halogen, and
R⁵ represents any one of the following:

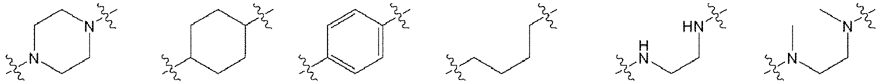

9. A pharmaceutical composition for analgesia through inhibition of an alpha-2 adrenergic receptor, comprising, as an active ingredient,
at least one selected from the group consisting of the compound represented by Formulae (I) to (VIII) according to any one of claims 3 to 8, the prodrug thereof, the pharmaceutically acceptable salt thereof, and a combination thereof.

10. A method for inhibiting an alpha-2 adrenergic receptor, comprising
administering at least one selected from the group consisting of the compound represented by Formulae (I) to (VIII) according to any one of claims 3 to 8, the prodrug thereof, the pharmaceutically acceptable salt thereof, and a combination thereof.

11. A method for relieving pain through inhibition of an alpha-2 adrenergic receptor, comprising
administering at least one selected from the group consisting of the compound represented by Formulae (I) to (VIII) according to any one of claims 3 to 8, the prodrug thereof, the pharmaceutically acceptable salt thereof, and a combination thereof.

12. Use of at least one selected from the group consisting of the compound represented by Formulae (I) to (VIII) according to any one of claims 3 to 8, the prodrug thereof, the pharmaceutically acceptable salt thereof, and a combination thereof, for inhibiting an alpha-2 adrenergic receptor.

13. Use of at least one selected from the group consisting of the compound represented by Formulae (I) to (VIII) according to any one of claims 3 to 8, the prodrug thereof, the pharmaceutically acceptable salt thereof, and a combination thereof, for relieving pain through inhibition of an alpha-2 adrenergic receptor.
